# EUROPEAN PATENT APPLICATION

(11) **EP 1 953 662 A1**
(43) Date of publication of application: **06.08.2008**
(21) Application number: 08001684.3
(22) Date of filing: 30.01.2008
(51) Int. Cl.: G06F 19/00

(54) **Molecular histology**

(30) Priority: 02.02.2007 EP 07002338
(71) Applicant: Roche Diagnostics GmbH, 68305 Mannheim (DE); F.HOFFMANN-LA ROCHE AG, 4070 Basel (CH)
(72) Inventor: Renzing, Jochen, 82393 Iffeldorf (DE); Fuerst, Roderic, 62128 Village Neuf (FR); Gutekunst, Martin, 82390 Eberfing (DE)

(57) **Abstract**

The present invention provides modified digital images of a multicellular sample using molecular profiles of cells from said multicellular sample for a molecular histological analysis.

## Description

### Background of the Invention

Recent technological advances allow the precise measurement of single-cell molecular profiles. For instance, Bengtson, M., et al. (Genome Research 15 (2005) 1388-1392) have studied the expression of multiple genes in individual mouse pancreatic islet cells by reverse transcriptase quantitative real-time PCR (RT-QPCR). This technique affords superior sensitivity, accuracy, and dynamic range compared with that of alternative methods for gene expression analysis. Schlieben et al. have previously described methods for the mRNA isolation from individual limited cell samples and single cells (Technical Note TN41000-003 2004, Bio-Nobile Oy). In an attempt to enhance the processing of morphological data derived from microscopic imaging, Kotsianti et al. have described a workflow from image acquisition of morphological data to pathological classification of tissue (US 2005/0165290). Further, there are descriptions available of how a combination of biomarker data and bio-image data may be used to derive a predictive diagnosis (Saidi et al., US 2005/0108753).

Options to isolate small numbers of purified cells from complex cellular samples such as micromanipulation, fluorescence-activated cell sorting or laser microdissection are e.g. described in the following review articles: Burgemeister, R., J. Histochem. Cytochem. 53 (2005) 409-412, and Baech, J. and Johnsen, H.E., Stem Cells. 18 (2000) 76-86.

However, presently there are no seamless data processing solutions available to combine the morphological information derived from microscopic imaging of biological samples with subsequently derived information on the molecular state of the processed samples, in particular, molecular data derived on the single cell level.

The disparity between the ability to measure and the desire to make biological or diagnostic conclusions calls out for a new approach for data processing and data handling in this context.

In general, a pathologist or researcher uses a visual assessment for the determination of disease status or morphological status of a cell containing sample. Therefore, in order to enhance cell analysis and speed up sample throughput a computer aided system for assessment and diagnosis is desirable enabling an automation of the analysis process that converts digital images and primary molecular data into summary data for use by a researcher or pathologist. In other words, a method is needed that integrates molecular data obtained by cell analysis with morphological data of cell images in one seamless process.

### Summary of the Invention

The present invention provides a new level of data quality for an improved diagnostic output of the examination of histo-pathological samples by providing a virtual digital image of predictive or diagnostic value.

To solve the above mentioned problem this invention provides a method, a computer program, a database and a system to improve data processing and data handling for molecular histological analysis of a multicellular sample to gain a new level of data quality by retaining the contextual morphological information of measured molecular cell values.

In other words, the results of measurements performed with cells from a multicellular sample are combined with morphological information contained in digital images. The results should be integrated into the image in such a way that a good impression of the morphology and histological status of the cells is retained for examination by the user. This integration allows the visualization of cellular quantities in the location of individual cells and therefore, to directly compare cells of the multicellular sample.

One aspect of the present invention is a system to perform a molecular histological analysis of a multicellular sample, comprising
a) an optical device to produce a digital image of said multicellular sample,
b) a computer means to select a region of interest within said digital image corresponding to one cell or a number of cells within said multicellular sample,
c) an isolation device to isolate said cell or said number of cells selected in step b) from said multicellular sample,
d) an analysis device to perform a biochemical analysis and to produce a molecular profile of said isolated cell or said number of isolated cells, said molecular profile comprises a plurality of molecular values,
e) a software application to store said digital image and said molecular profile in such a way that said molecular profile is linked to the corresponding selected region of interest within said digital image, and
f) a visualizing means to present a modified digital image comprising said digital image produced in step a) together with one or more of said molecular values produced in step d),
wherein said molecular histological analysis is based on said modified digital image.

Throughout the present invention a molecular histological analysis is the analysis of an multicellular sample with respect to morphological as well as molecular aspects. A multicellular sample is an assembly of cells, such as in a tissue sample or a cell culture.

In order to gain the morphological information of the multicellular sample the system of the present invention has an optical device to record a digital image of the multicellular sample. As optical device all kinds of devices known to someone skilled in the art are suitable, such as devices to perform light microscopy, contrast microscopy, confocal microscopy or fluorescence microscopy.

Within the recorded digital image the system selects cells that are of further interest and defines a region within said digital image corresponding to the cell of interest. This region within the recorded digital image is called region of interest (ROI) throughout the present invention. This ROI is designed by the computer means as the exact contour of the cell having a complex shape or as a simple shape (like a circle or square) comprising the cell and part of its surrounding. Alternatively, such a ROI can also be defined to include more than one cell.

The selection of the ROI can be initiated by manual input of the user indicating the cells of interest or automatically by the computer means using a pattern recognition algorithm. Using such a pattern recognition algorithm, it is possible that this computer means not only selects the ROI but also identifies the cells of interest.

The selected ROIs are than used by the isolation device to isolate said cell or said number of cells from said multicellular sample that correspond to the selected regions within the digital image. The phrase number of cells is used throughout the present invention to emphasize that it is not necessary for all applications of the present system to isolate a plurality of single cells from the multicellular sample, but that groups of neighboring cells can be sufficient. Afterwards, such a group of neighboring cells from the multicellular sample is further processed together in order to produce a single molecular profile.

Subsequently, the isolated cell or cells of each ROI are placed in separate storages for further processing. Said storage can be e.g. a separate vessel for each ROI or a multiwell plate, whereas each well contains the cell or cells from one ROI.

The analysis device is necessary to perform a biochemical analysis with the isolated cells in order to produce molecular profiles. For this purpose, the analysis device of the present invention is able to perform a separate biochemical analysis for each storage containing cells from one ROI.

The analysis device produces molecular profiles of cells isolated from said multicellular sample. This implies that a lysis of the cells is required to reach the molecular constituents such as nucleic acids and proteins. The phrase molecular profile is used throughout the present invention to emphasize that more than one molecular constituent of the respective cells is analyzed. Therefore, throughout the present invention a molecular profile comprises a plurality of molecular values obtained by isolating a cell or a number of cells from a multicellular sample. Such molecular values of the corresponding molecular constituent can be e.g. the concentration, the absolute amount, a relative ratio with respect to a control or reference or a physical quantity as qualitative measure.

The gained digital image and molecular profile are processed by a software application. Throughout the present invention said software application must enable that the digital images are stored together with the molecular profile in such a way that each molecular profile is linked to the selected region of interest within said digital image corresponding to the cells that were isolated to obtain said molecular profile. In general, there are at least two possible alternatives to provide such a software application, namely a data base application or an image-based application.

Finally, the system according to the present invention comprises a visualizing means to present modified digital images to the user. A modified digital image is based on the original digital image recorded by the optical device that is modified with a certain number of molecular values in such a way that said molecular values are visualized within the digital image and that each molecular value is clearly linked to the region of interest within the modified digital image corresponding to its cellular origin.

Another aspect of the present invention is a method to perform a molecular histological analysis of a multicellular sample, comprising
a) obtaining a digital image of said multicellular sample,
b) selecting a region of interest within said digital image corresponding to one cell or a number of cells within said multicellular sample,
c) isolating said cell or said number of cells selected in step b) from said multicellular sample,
d) performing a biochemical analysis to produce a molecular profile of said isolated cell or said number of isolated cells, said molecular profile comprises a plurality of molecular values, and
e) producing a modified digital image comprising said digital image obtained in step a) together with one or more of said molecular values produced in step d) to perform said molecular histological analysis.

Yet another aspect of the present invention is a method to identify the location of characteristic cells within a multicellular sample in order to perform a molecular histological analysis, said method comprises
a) providing a digital image of said multicellular sample together with molecular profiles of cells or number of cells within said multicellular sample, each molecular profile comprises a plurality of molecular values and is linked to a region of interest within said digital image corresponding to the respective cell or number of cells,
b) highlighting in a modified digital image those cells within said multicellular sample that fulfill a defined molecular criteria,
wherein said molecular profiles are obtained by isolating a cell or a number of cells from said multicellular sample.

This embodiment of the present invention refers to a method that enables a user to identify those cells within a multicellular sample that fulfill a certain molecular criteria by examination a modified digital image. Cells that fulfill such a molecular criteria are named characteristic cells throughout the present invention and the identification of said characteristic cells is necessary to perform the molecular histological analysis of the multicellular sample.

There are many different molecular criteria that are of interest for a molecular histological analysis of a multicellular sample. For example, all cells within the multicellular sample can be highlighted that exceed a certain threshold with respect to the concentration of a cellular constituent. It can also desirable to visualize more complex molecular criteria such as a concentration relative to a reference or control, a concentration criteria combined with a distance demand or a molecular amount combined with a morphological criteria.

Using the method to identify the location of characteristic cells according to the present invention, a plurality of histological hypotheses can be tested based on a digital image of a multicellular sample and a certain number of molecular profiles of cells of this multicellular sample.

Still another aspect of the present invention is a computer program to generate a plurality of modified digital images from the digital image of a multicellular sample, said digital images are modified with one or more molecular value of respective cells or number of cells within said multicellular sample, said computer program comprises
a) loading said digital image of said multicellular sample and a plurality of molecular values of cells or number of cells from said multicellular sample, each molecular value is linked to a region of interest within said digital image corresponding to the respective cell or number of cells, and
b) generating a modified digital image comprising said digital image and a number of selected molecular values,
wherein said molecular values are obtained by isolating a cell or a number of cells from said multicellular sample.

Such a computer program generates a plurality of modified digital images in order to perform e.g. a molecular histological analysis that is based on said modified digital image.

This computer program according to the present invention enables a user to generate modified digital images that highlight those cells within a multicellular sample that fulfill a certain user-defined molecular criteria. This modified digital image can be used e.g. to verify histological hypotheses.

Moreover, an aspect of the present invention is a method for managing molecular histological experiments of a multicellular sample, said method comprises
a) loading a digital image of said multicellular sample into a software application,
b) defining regions of interest within said digital image that are linked to corresponding positions of cells within said multicellular sample, and
c) loading a plurality of molecular profiles of cells or number of cells from said multicellular sample into said software application, each molecular profile comprises a plurality of molecular values,
wherein said software application is structured such that said plurality of molecular profiles loaded in step c) are linked to the corresponding region of interest defined in step b) within said digital image loaded in step a) and
wherein said molecular profiles are obtained by isolating a cell or a number of cells from said multicellular sample.

In addition, the present invention relates to a computer program to identify the location of characteristic cells within a multicellular sample in order to perform a molecular histological analysis, said computer program comprises
a) a first application to load a digital image of multicellular sample, to define regions of interest within said digital image that are linked to corresponding positions of cells within said multicellular sample and to save said digital image together with said regions of interest as a first image file,
b) a second application to combine said first image file with a plurality of molecular profiles comprising a plurality of molecular values and to save said first image file together with said plurality of molecular profiles as a second image file, wherein said plurality of molecular profiles are linked to the corresponding region of interest within said digital image, and
c) a third application to extract modified digital images from said second image file according to user-defined molecular criteria, wherein said modified digital images highlight cells that fulfill said user-defined molecular criteria,
wherein said molecular profiles are obtained by isolating a cell or a number of cells from said multicellular sample.

The computer program according to this embodiment of the present invention comprises three different applications performing parts of the overall computer program.

The first application is an imaging application to load a digital image and to define ROIs within said digital image. As mentioned before, the ROI can be the exact contour of the cell having a complex shape or a simple shape (like a circle or square) comprising the cell and part of its surrounding. Alternatively, such a ROI can include more than one cell. The definition of the ROIs can be initiated by a manual input of the user of the system indicating the cells of interest or automatically by a pattern recognition algorithm. For the alternative of manual input, the first application requires the possibility to display the digital image for examination by the user. Moreover, said first application has a saving function to save the digital image together with the defined ROIs, whereas the file comprising the digital image and the defined ROIs is called a first image file.

The second application is able to add to said first image file a plurality of molecular profiles and to save both components together as a second image file. The second application is designed to link the molecular profiles to the corresponding ROIs representing the cells of the multicellular sample that provided said molecular profiles.

The third application is necessary to extract modified digital images from the second image file. As mentioned before, a modified digital image is based on the original digital image modified with a certain number of molecular values, whereas each modified digital image is created according to a user-defined criteria. Different kinds of criteria were mentioned before and comprise e.g. cells with a concentration of a cellular constituent above a certain threshold or cells with a certain relative concentration.

The present invention also relates to a database for managing molecular histological experiments of a multicellular sample, said database comprises
a) a cell table comprising cell numbers together with the corresponding position within said multicellular sample,
b) a molecular value table comprising molecular profiles of cells within said multicellular sample, each molecular profile comprises a plurality of molecular values, and
c) a digital image table comprising digital images of said multicellular sample and coordinates of cells within said digital image,
wherein said cell table is linked with said molecular value table and with said digital image table such that each cell of said multicellular sample is linked to the corresponding coordinates within the respective digital image and to the corresponding molecular profile,
wherein said database is designed such that a modified digital image based on a digital image from said digital image table highlighting cells fulfilling a plurality of user-defined molecular criteria is producible and
wherein said molecular profiles are obtained by isolating a cell or a number of cells from said multicellular sample.

The database according to this embodiment of the present invention comprises at least three substructures, namely a cell table, a molecular value table and a digital image table that are linked to each other in order to produce modified digital images based on a digital image from said digital image table and a certain number of molecular values from said molecular value table. This database is structured such that a plurality of modified digital images can be extracted upon defining a certain molecular criteria.

The cell table summarizes information about each cell of interest that is used for the molecular histological experiment, namely the position of said cell within the multicellular sample together with an assigned identification number and if necessary, an analysis number (e.g. a well number) for the subsequent biochemical analysis.

The molecular value table comprises all molecular values obtained by the biochemical analysis, whereas all molecular values belonging to a respective cell are grouped as a molecular profile.

In the digital image table the digital images of the multicellular sample are stored together with coordinates that define the ROI corresponding to the cells of interest within the respective digital image.

### Detailed Description of the Invention

One aspect of the present invention is a system to perform a molecular histological analysis of a multicellular sample, comprising
a) an optical device to produce a digital image of said multicellular sample,
b) a computer means to select a region of interest within said digital image corresponding to one cell or a number of cells within said multicellular sample,
c) an isolation device to isolate said cell or said number of cells selected in step b) from said multicellular sample,
d) an analysis device to perform a biochemical analysis and to produce a molecular profile of said isolated cell or said number of isolated cells, said molecular profile comprises a plurality of molecular values,
e) a software application to store said digital image and said molecular profile in such a way that said molecular profile is linked to the corresponding selected region of interest within said digital image, and
f) a visualizing means to present a modified digital image comprising said digital image produced in step a) together with one or more of said molecular values produced in step d),
wherein said molecular histological analysis is based on said modified digital image.

An important issue of the system according to the present invention is the selection of the region of interest (ROI) by the computer means in step b). As mentioned before, there are different alternatives to define ROIs within the digital image of the optical device, e.g. to define the ROI as the contour of the cell of interest or as a circle or square spanning the expanse of the cell of interest.

In a preferred embodiment of the present invention, said region of interest is defined by two or more coordinates.

The simplest way to define a ROI requires only two coordinates defining the center of a circle spanning the expanse of the cell of interest. On the other hand, a ROI expressing the contour of the cell requires a large number of coordinates.

In another preferred embodiment of the present invention, said optical device is disposed to perform light microscopy.

As mentioned before a plurality of different optical devices that are known to someone skilled in the art are suitable for the present invention. Examples for devices to perform light microscopy are a commercial light microscope, a contrast microscope or a confocal microscope. Using light microscopy to produce digital images of the multicellular sample may result in the necessity to treat the sample with compounds that enhance the optical contrast. Such contrast enhancers are known to someone skilled in the art. The most widely used staining technique is that of Haematoxylin and Eosin, commonly called H&E. In this method the nuclei of cells is stained by the haematoxylin whilst the cytoplasm is colored by the eosin. This technique in various forms has been around for over one hundred years. Waldeyer was the first person to try staining histological section with an extract of Logwood in 1863 (Waldeyer, W., Zeitschr. f. rat. Med. (Dritte R.) Bd. XX (1863) 193-256). The stain haematoxylin is not a dye, but develops coloring properties on oxidation to haematin. This substance haematin has little or no affinity for tissue elements and requires an inorganic ion to act as a "go between" (called a mordant) between the dye and the tissue. The mordant may be incorporated into the haematoxylin dye bath, the most common method. Or one may pre-treat the tissue with the metal salt and then stain by the haematoxylin. Eosin is an acid dye, which requires an acidic environment to work. In solution the dye molecule is negatively charged and thus attaches to positive site in the tissue by salt bridges.

Moreover, it is of course necessary to use an optical device that provides a magnification of the cellular structures.

In yet another preferred embodiment of the present invention, said optical device is a fluorescence optics, preferably a fluorescence microscope.

Using fluorescence microscopy provides advantages compared to light microscopy such as resolution, multiplexing or contrast. Moreover, established analysis techniques for tissue and cell cultures such as FISH (Fluorescence in-situ hybridization) or fluorescent labeled antibodies (for tissue (immuno histochemistry) or cell cultures (immuno cytochemistry)) can be used to gain some molecular information already during the step of image recording.

In an also preferred embodiment of the present invention, said isolation device is a microdissection device, preferably a laser assisted microdissection device.

In yet another preferred embodiment of the present invention, said isolation device is a manual, microdissection device, preferably a micropipetting device.

Devices and methods that are suitable for the present invention to isolate single cells from cell cultures or tissue samples are known to someone skilled in the art. Options to isolate small numbers of purified cells from complex cellular samples such as micromanipulation, fluorescence-activated cell sorting or laser microdissection are e.g. described in the review article of Player, A., et al. (Expert. Rev. Mol. Diagn. 4 (2004) 831-840). Laser microdissection devices are commercially available e.g. from P.A.L.M. Microlaser Technologies GmbH, Bernried, Germany.

A preferred system according to the present invention is a system, wherein said number of isolated cells in step c) comprises less than 100, preferably less than 10, most preferably less than 5 cells.

Depending on the application of the system and the properties of the used analysis device different requirements with respect to the number of cells per isolation can apply. As mentioned before, a number of cells that are isolated in a single isolation step are always a group of neighboring cells from the same part of the multicellular sample. In general, it is of course preferred to isolate as little cells as possible, ideally just one cell in order to gain a maximum of information about the multicellular sample. There are commercially available microdissection devices able to isolate single cells from tissue, e.g. from P.A.L.M. Microlaser Technologies GmbH (Bernried, Germany).

Another preferred system according to the present invention is a system, wherein said analysis device is capable of performing a downstream analysis process, preferably said analysis device is an immunoassay, a mass spectrometer, a nucleic acid sequencing, a nucleic acid hybridization assay, a surface plasmon resonance, or gel electrophoresis, more preferably said analysis device is a PCR device, most preferably said analysis device is a real-time PCR device.

Throughout the present invention the phrase downstream analysis process summarizes all processes that are suitable to gain further information about the isolated cells on a molecular level. Within the present invention said further information on a molecular level is obtained after isolation of the cells in an external biochemical analysis. But note that this does not exclude the possibility to gain additional molecular cell information already during image recording based on labeled antibodies or in-situ hybridization.

From the group of suitable downstream analysis processes to gain further molecular information on basis of nucleic acids, a PCR device is especially preferred, because of the small amount of nucleic acid that in general is obtained from only a number of isolated cells. Moreover, using a real-time PCR provides the desired amplification and the required analysis in a single process. Additionally, real-time PCR offers multiplexing capabilities that are helpful to gain a molecular profile of the cells. One biochemical analysis to which this invention can be applied is the quantification of mRNA derived from individual cells isolated from a multicellular sample.

Yet another preferred system according to the present invention is a system, wherein said software application is capable of storing a plurality of molecular profiles, each linked to the corresponding selected region of interest within said digital image.

Since the system of the present invention is applicable for the molecular histological analysis of a multicellular sample, it is necessary that the software application can handle multiple molecular profiles that are linked to different regions within a single digital image.

In general, there are at least two alternatives to store a digital image together with multiple molecular profiles and their allocation to respective regions of interest, namely a database or an image file.

In a more preferred embodiment of the present invention, said software application is a database, an image-based application or a combination thereof.

In another more preferred embodiment of the present invention, said image-based application is a TIFF-application.

The TIFF-format allows storing additive information and image overlays by block structure and headers describing the block content. To use the TIFF-format for the present invention a software module has to be used to read the TIFF-format and to display the image with a data overlay.

The software application annotates an object in the image by an identifier which correlates to the position of the object and/or its morphological structure and should also include the name or number of the respective image. This identifier is also linked to molecular profiles of the object corresponding to said identifier that are stored in said block structure or e.g. in a relational database.

Finally, the software application can display the image together with the molecular profiles of the objects. This can iteratively be done by the user marking objects defined by the position and/or the morphological structure that correspond to an identifier (including the image name or image number). The software can now display the molecular profile of the object within the image by reading data corresponding to the identifier from the database or the block structure. This workflow also works for displaying information of several objects in parallel.

In a preferred embodiment of the present invention, said visualizing means presents digital images together with molecular values from a plurality of selected regions of interest within said digital image.

As mentioned before, the system of the present invention is applicable for the molecular histological analysis of a multicellular sample and therefore, it is necessary that the visualizing means can handle a plurality of molecular values belonging to different regions of interest.

In another preferred embodiment of the present invention, said visualizing means presents digital images together with molecular values chosen by a user.

In order to indicate the required molecular values within the modified digital image several alternatives exist. First of all, a molecular value can be displayed as a numerical value at the position of the respective cell, said numerical value can be e.g. the concentration of said molecular compound. Alternatively, the molecular value can be displayed by color, e.g. in such a way that a concentration is displayed at the position of the respective cell according to a color scale ranging from white to black or from white to red. Using different color scales a certain number of different concentration can be displayed simultaneously. Moreover, single colors can be used to mark those cells that fulfill certain molecular criteria, e.g. all cells that have protein A and protein B are marked red. Also the intensity of a single color can be used to display molecular values.

With this kind of visualization the present invention is suitably applied to a plurality of user-defined processes e.g. the process of measuring populations of cells that fulfill different molecular criteria based on molecular species that are derived from individual cells contained in biological samples.

In an also preferred embodiment of the present invention, said multicellular sample is a tissue sample, a cell culture sample, a biopsy sample or a cytological sample.

Such tissue samples or cell culture samples are typically used for research applications. A biopsy sample such as a histo-pathological sample or a fine needle aspirate are also used for medical or diagnostic applications. For other medical or diagnostic applications of the system of the present invention said multicellular sample can be a cytological sample, such as a blood smear, a bucchal swap or a PAP smear. Alternatively, such a cytological sample can consist of stem cells for implantation purposes. Moreover, said multicellular sample can be a cytological sample derived from an amniocentesis.

A preferred system according to the present invention is a system, wherein said molecular profiles are nucleic acid profiles.

Such a nucleic acid profile can be a nucleic acid expression profile a nucleic acid variation profile, nucleic acid methylation profile or other nucleic acid modification profile.

Another preferred system according to the present invention is a system, wherein said molecular profiles are protein profiles.

Such a protein profile is e.g. a protein expression profile a post-translational protein modification profile or a protein activity profile.

Another preferred system according to the present invention is a system, wherein said molecular profiles are profiles of metabolytes and other endogenous and exogenous small molecular entities (sme) including signal transduction molecules and hormones

Such a metabolite profile is e.g. a profile of endogenous physiological or pathophysiological metabolytes or a profile of xenobiotic metabolytes (metabolytes of pharmacologically or txocologically relevant exogenous substances).

Yet another preferred system according to the present invention is a system, wherein said molecular histological analysis is performed automatically using a prediction algorithm together with said digital image and said molecular profiles.

The system of the present invention may further be configured such that input for computer aided algorithms is provided allowing an automated analysis of the modified digital images created from the multicellular sample image combined with molecular profiles of multiple molecular species to generate additional interpretive images, medical data, medial statistics or medical reports of predictive value or diagnostic value.

Moreover, the system according to the present invention may be further configured to convert the primary data of several histological samples or cytological specimen into a computational, three-dimensional model of an organ or organism. A combination of image data of the organ, molecular signature(s) of cell(s), and phenotype of cell contained in the input sample may enable computational quantitative analysis to provide an assessment for disease status of a histological sample or cytological specimen.

Additionally, the system according to the present invention may further be configured to be capable of processing, interrogating, and/or communicating with a computer network, serving as input for a medical decision support system.

Another aspect of the present invention is a method to perform a molecular histological analysis of a multicellular sample, comprising
a) obtaining a digital image of said multicellular sample,
b) selecting a region of interest within said digital image corresponding to one cell or a number of cells within said multicellular sample,
c) isolating said cell or said number of cells selected in step b) from said multicellular sample,
d) performing a biochemical analysis to produce a molecular profile of said isolated cell or said number of isolated cells, said molecular profile comprises a plurality of molecular values, and
e) producing a modified digital image comprising said digital image obtained in step a) together with one or more of said molecular values produced in step d) to perform said molecular histological analysis.

The method according to the present invention produces modified digital images based on an original digital image of a multicellular sample (e.g. a contrast enhanced photograph using a magnification or a fluorescence image) and molecular values that are obtained by an external biochemical analysis of isolated cells. Options to isolate cells and perform biochemical analysis were already described before.

A preferred method according to the present invention is a method wherein steps b) to d) are repeated for different regions of interest.

Another preferred method according to the present invention is a method wherein steps b) to d) are repeated until all cells of the multicellular sample are analyzed.

In a preferred method according to the present invention a plurality of modified digital images are producible, each with a different molecular value or with different groups of molecular values.

There are of course many ways to modify a digital image in order to provide support for a molecular histological analysis of a multicellular sample. Said modified digital images can help to answer questions like e.g., where in the multicellular sample are those cells that have an overexpression of a certain gene or are there cells with a certain gene expression that have a certain distance from each other or where are cells that have an overexpression of a certain gene while an other gene is downregulated. To answer such kind of questions, it can be necessary to indicate one or more molecular values within the modified digital image.

In another preferred method according to the present invention, said modified digital images contain said molecular values as numerical values or indicated by color.

In yet another preferred method according to the present invention, said modified digital images comprise said digital image together with molecular values from different regions of interest.

In an also preferred method according to the present invention, said molecular values of said modified digital images are individually selected for visualization by a user.

The different alternatives to visualize different molecular values within a digital image according to user-defined criteria were already described before.

A preferred method according to the present invention is a method, wherein said biochemical analysis is a gene expression analysis based on PCR amplification, preferably a real-time PCR.

As mentioned before, from the group of suitable downstream analysis processes to gain further molecular information of the cells, a PCR amplification is especially preferred, because of the small amount of nucleic acid that can be obtained from only a number of isolated cells. Moreover, using a real-time PCR offers multiplexing capabilities and provides the desired amplification and the required analysis in a single process.

Another preferred method according to the present invention is a method, wherein said molecular profiles are nucleic acid profiles, preferably gene expression profiles.

Using PCR as downstream analysis process it is possible to analyze gene expression of cells in terms of quantification of mRNA derived from individual cells isolated from a multicellular sample. Depending on the complexity of the desired molecular histological analysis a certain number of genes need to be screened within the multicellular sample.

A more preferred method according to the present invention is a method, wherein said PCR amplifies more than 5, preferably more than 10, most preferably more than 100 genes.

Note that in principle the expression of all genes that are analyzed and summarized in the respective molecular profile can be displayed in one modified digital image using color, intensity and/or numerical values. But since the value of such a complex digital image is questionable, it is desirable to produce a number of different modified digital images displaying only a certain amount of all analyzed genes.

Yet another preferred method according to the present invention is a method, wherein said molecular profiles are protein profiles.

Alternatively to downstream processes on basis of nucleic acid analysis it is of course possible to study the cells of the multicellular sample with respect to their protein content resulting in a protein profile of the isolated cells.

A more preferred method according to the present invention is a method, wherein said biochemical analysis is an immunoassay.

Alternatively to a process where the molecular data on protein profiles is obtained by conventional in-situ staining, such as immuno-histochemistry or immuno-cytochemistry, a process can be conceived where, following (single) cell isolation, the cell content is transferred to separate reaction vessels (or surfaces) for subsequent analysis of one or more molecular properties of one or more protein species. A preferred analytical approach for this purpose could be an ELISA technique or a protein expression array technique. In order to circumvent technical difficulties in multiplex-protein analysis, such as limitations in sensitivity such an analytical approach could be based for example on methods like immuno-PCR.

Another more preferred method according to the present invention is a method, wherein said immunoassay detects more than 5, preferably more than 10, most preferably more than 100 proteins.

Yet another aspect of the present invention is a method to identify the location of characteristic cells within a multicellular sample in order to perform a molecular histological analysis, said method comprises
a) providing a digital image of said multicellular sample together with molecular profiles of cells or number of cells within said multicellular sample, each molecular profile comprises a plurality of molecular values and is linked to a region of interest within said digital image corresponding to the respective cell or number of cells,
b) highlighting in a modified digital image those cells within said multicellular sample that fulfill a defined molecular criteria,
wherein said molecular profiles are obtained by isolating a cell or a number of cells from said multicellular sample.

As mentioned before, this embodiment of the present invention refers to a method that enables a user to identify characteristic cells within a multicellular sample that fulfill a certain molecular criteria by examination a modified digital image. For this purpose, each cell of the multicellular sample corresponds to a so-called region of interest within the digital image such that a defined position or area exist for each cell in order to modify the digital image at said position or area with the respective molecular values.

In a preferred method to identify the location of characteristic cells according to the present invention, said region of interest of the respective cells is defined by two or more coordinates.

Yet another preferred method to identify the location of characteristic cells according to the present invention is a method, wherein said highlighting is performed by numerical values or by color.

The alternatives to define the ROIs within the digital image as well as the subsequent isolation of the corresponding cells within the multicellular sample and the indication of molecular values within modified digital images were described in detail before.

In yet another preferred method to identify the location of characteristic cells according to the present invention, said molecular profiles are nucleic acid profiles, preferably gene expression profiles.

A preferred method to identify the location of characteristic cells according to the present invention is a method, wherein said molecular criteria is the expression level of a gene or the expression level of a associated group of genes.

Another preferred method to identify the location of characteristic cells according to the present invention is a method, wherein said molecular profiles are protein profiles.

A more preferred method to identify the location of characteristic cells according to the present invention is a method, wherein said molecular criteria is the concentration of a protein.

As mentioned before, the molecular histological analysis of the multicellular sample are preferably based on the measurement of the nucleic acid or protein content of the isolated cells.

Still another aspect of the present invention is a computer program to generate a plurality of modified digital images from the digital image of a multicellular sample, said digital images are modified with one or more molecular value of respective cells or number of cells within said multicellular sample, said computer program comprises
a) loading said digital image of said multicellular sample and a plurality of molecular values of cells or number of cells from said multicellular sample, each molecular value is linked to a region of interest within said digital image corresponding to the respective cell or number of cells, and
b) generating a modified digital image comprising said digital image and a number of selected molecular values,
wherein said molecular values are obtained by isolating a cell or a number of cells from said multicellular sample.

Preferably, said computer program generates said plurality of modified digital images in order to perform a molecular histological analysis that is based on said modified digital images.

The computer program to generate a plurality of modified digital images according to the present invention enables a user to generate modified digital images that highlight those cells within a multicellular sample that fulfill a certain user-defined molecular criteria. This modified digital image can be used e.g. to verify histological hypotheses.

For this purpose it is essential that the computer program is able to link molecular profiles to defined regions of a digital image while loading said digital image and said molecular profile. The defined regions of a digital image in general correspond to a single cell or a group of neighboring cells and said region is called a region of interest (ROI).

In a preferred computer program according to the present invention, said region of interest of the respective cells is defined by one or more coordinates.

In another preferred computer program according to the present invention, said selected molecular values on said modified digital images are indicated as numerical values or indicated by color.

The alternatives for defining a ROI and for indicating molecular values were already described in detail before.

In yet another preferred computer program according to the present invention, a threshold of a certain molecular value of interest is selected and all cells are highlighted within said modified digital image that exceed or that not exceed said threshold of said molecular value of interest.

In this special embodiment of the computer program according to the present invention, the criteria that is indicated within the modified digital image is a threshold test for a certain molecular value. Such a threshold test indicates e.g. all cells within the multicellular sample with an up or down regulation of a gene of interest, whereas the threshold is the normal expression of said gene.

Another preferred computer program according to the present invention is a computer program, wherein said molecular values are nucleic acid expression values.

Yet another preferred computer program according to the present invention is a computer program, wherein said molecular values are protein expression values.

Moreover, an aspect of the present invention is a method for managing molecular histological experiments of a multicellular sample, said method comprises
a) loading a digital image of said multicellular sample into a software application,
b) defining regions of interest within said digital image that are linked to corresponding positions of cells within said multicellular sample, and
c) loading a plurality of molecular profiles of cells or number of cells from said multicellular sample into said software application, each molecular profile comprises a plurality of molecular values,
wherein said software application is structured such that said plurality of molecular profiles loaded in step c) are linked to the corresponding region of interest defined in step b) within said digital image loaded in step a) and
wherein said molecular profiles are obtained by isolating a cell or a number of cells from said multicellular sample.

In a preferred method for managing molecular histological experiments according to the present invention, said region of interest is defined by one or more coordinates.

In another preferred method for managing molecular histological experiments according to the present invention, said molecular profiles are obtained by isolating a cell or a number of cells from said multicellular sample.

In yet another preferred method for managing molecular histological experiments according to the present invention, said molecular profiles are nucleic acid profiles, preferably gene expression profiles.

A preferred method for managing molecular histological experiments according to the present invention is a method, wherein said molecular profiles are protein profiles.

Another preferred method for managing molecular histological experiments according to the present invention is a method, wherein a molecular histological analysis of said multicellular sample is performed.

As mentioned before, a molecular histological experiment is the analysis of an multicellular sample with respect to morphological as well as molecular aspects.

Yet another preferred method for managing molecular histological experiments according to the present invention is a method, wherein a modified digital image of said multicellular sample is produced in order to perform a histological analysis of said multicellular sample.

After the molecular histological experiment is performed, the obtained molecular values are used to produce one or more modified digital images, said modified digital images are the basis for a histological analysis of said multicellular sample.

An also preferred method for managing molecular histological experiments according to the present invention is a method, wherein said software application is a database, an image-based application or a combination thereof.

A more preferred method for managing molecular histological experiments according to the present invention is a method, wherein said image-based application is a TIFF-application.

The different software applications that are applicable throughout the present invention were already described in detail before.

In addition, the present invention relates to a computer program to identify the location of characteristic cells within a multicellular sample in order to perform a molecular histological analysis, said computer program comprises
a) a first application to load a digital image of multicellular sample, to define regions of interest within said digital image that are linked to corresponding positions of cells within said multicellular sample and to save said digital image together with said regions of interest as a first image file,
b) a second application to combine said first image file with a plurality of molecular profiles comprising a plurality of molecular values and to save said first image file together with said plurality of molecular profiles as a second image file, wherein said plurality of molecular profiles are linked to the corresponding region of interest within said digital image, and
c) a third application to extract modified digital images from said second image file according to user-defined molecular criteria, wherein said modified digital images highlight cells that fulfill said user-defined molecular criteria,
wherein said molecular profiles are obtained by isolating a cell or a number of cells from said multicellular sample.

Details of the three applications that are part of the computer program to identify the location of characteristic cells according to the present invention were already described in detail before.

In brief, the first application is an imaging application to load a digital image and to define ROIs within said digital image and to save the digital image together with defined ROIs as a first image file. The second application is able to add to said first image file a plurality of molecular profiles and to save both components together as a second image file. The third application is necessary to extract modified digital images from the second image file.

A preferred computer program to identify the location of characteristic cells according to the present invention is a program, wherein said region of interest is defined by one or more coordinates.

Another preferred computer program to identify the location of characteristic cells according to the present invention is a program, wherein said molecular profiles are nucleic acid profiles, preferably gene expression profiles.

Yet another preferred computer program to identify the location of characteristic cells according to the present invention is a program, wherein said molecular profiles are protein profiles.

In a preferred computer program to identify the location of characteristic cells according to the present invention, said modified digital image highlights cells by color.

In another preferred computer program to identify the location of characteristic cells according to the present invention, said computer program is an image-based application, preferably a TIFF-application.

The present invention also relates to a database for managing molecular histological experiments of a multicellular sample, said database comprises
a) a cell table comprising cell numbers together with the corresponding position within said multicellular sample,
b) a molecular value table comprising molecular profiles of cells within said multicellular sample, each molecular profile comprises a plurality of molecular values, and
c) a digital image table comprising digital images of said multicellular sample and coordinates of cells within said digital image,
wherein said cell table is linked with said molecular value table and with said digital image table such that each cell of said multicellular sample is linked to the corresponding coordinates within the respective digital image and to the corresponding molecular profile,
wherein said database is designed such that a modified digital image based on a digital image from said digital image table highlighting cells fulfilling a plurality of user-defined molecular criteria is producible and
wherein said molecular profiles are obtained by isolating a cell or a number of cells from said multicellular sample.

Details of the database components to managing molecular histological experiments of a multicellular sample according to the present invention were already described in detail before.

In brief, the database according to this embodiment of the present invention comprises a cell table summarizing information about each cell of interest that is used for the molecular histological experiment, a molecular value table comprising all molecular values obtained by the biochemical analysis and a digital image table storing digital images of the multicellular sample together with coordinates that define the ROIs.

In a preferred database according to the present invention, said molecular profiles are nucleic acid profiles, preferably gene expression profiles.

In another preferred database according to the present invention, said molecular profiles are protein profiles.

In yet another preferred database according to the present invention, said modified digital image highlights said cells by color.

The following figures are provided to aid the understanding of the present invention, the true scope of which is set forth in the appended claims. It is understood that modifications can be made in the procedures set forth without departing from the spirit of the invention.

### Description of the Figures

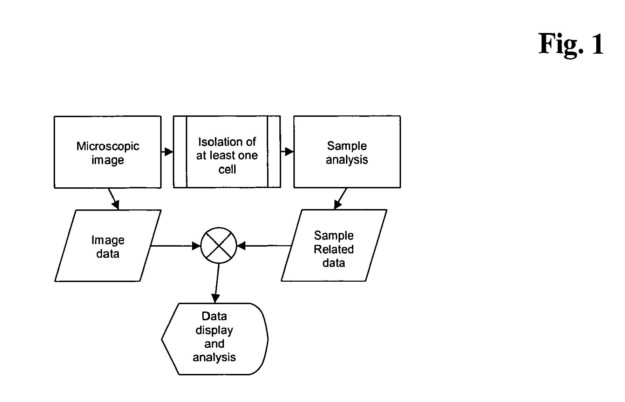

- Figure 1: Flow chart of the general method according to the present invention

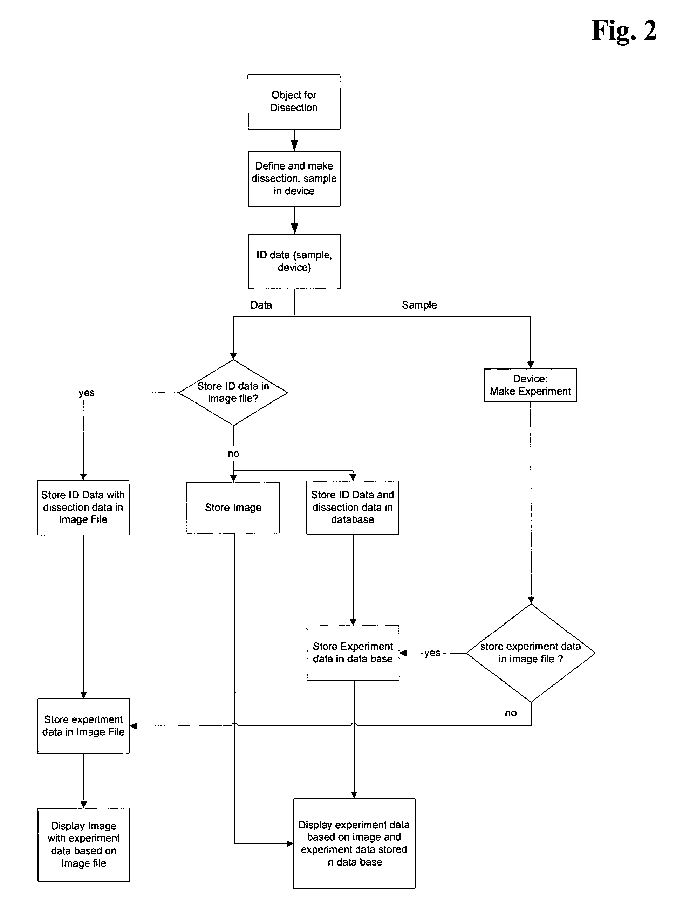

- Figure 2: A detailed flow chart of the method according to the present invention

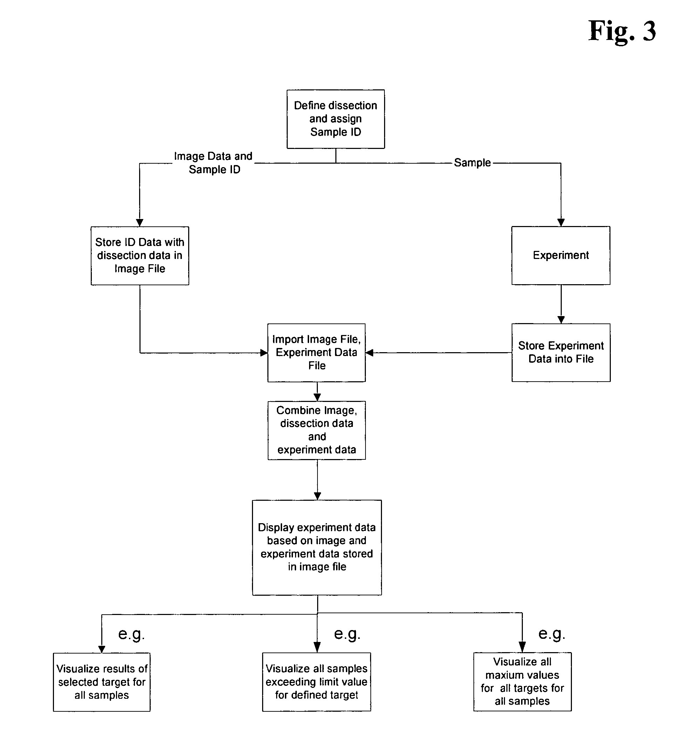

- Figure 3: Flow chart of one embodiment of the present invention

## Claims

1. A system to perform a molecular histological analysis of a multicellular sample, comprising
a) an optical device to produce a digital image of said multicellular sample,
b) a computer means to select a region of interest within said digital image corresponding to one cell or a number of cells within said multicellular sample,
c) an isolation device to isolate said cell or said number of cells selected in step b) from said multicellular sample,
d) an analysis device to perform a biochemical analysis and to produce a molecular profile of said isolated cell or said number of isolated cells, said molecular profile comprises a plurality of molecular values,
e) a software application to store said digital image and said molecular profile in such a way that said molecular profile is linked to the corresponding selected region of interest within said digital image, and
f) a visualizing means to present a modified digital image comprising said digital image produced in step a) together with one or more of said molecular values produced in step d),
wherein said molecular histological analysis is based on said modified digital image.

2. The system according to claim 1, wherein said optical device is a fluorescence optics, preferably a fluorescence microscope.

3. The system according to claims 1 to 2, wherein said isolation device is a microdissection device, preferably a laser assisted microdissection device.

4. The system according to claims 1 to 3, wherein said number of isolated cells in step c) comprises less than 100, preferably less than 10, most preferably less than 5 cells.

5. The system according to claims 1 to 4, wherein said analysis device is capable of performing a downstream analysis process, preferably said analysis device is an immunoassay, a mass spectrometer, a nucleic acid sequencing, a nucleic acid hybridization assay, a surface plasmon resonance, or gel electrophoresis, more preferably said analysis device a PCR device, most preferably said analysis device is a real-time PCR device.

6. The system according to claims 1 to 5, wherein said software application is a database, an image-based application or a combination thereof.

7. The system according to claims 1 to 6, wherein said multicellular sample is a tissue sample, a cell culture sample, a biopsy sample or a cytological sample.

8. The system according to claims 1 to 7, wherein said molecular profiles are nucleic acid profiles.

9. The system according to claims 1 to 7, wherein said molecular profiles are protein profiles.

10. The system according to claims 1 to 9, wherein said molecular histological analysis is performed automatically using a prediction algorithm together with said digital image and said molecular profiles.

11. A method to perform a molecular histological analysis of a multicellular sample, comprising
a) obtaining a digital image of said multicellular sample,
b) selecting a region of interest within said digital image corresponding to one cell or a number of cells within said multicellular sample,
c) isolating said cell or said number of cells selected in step b) from said multicellular sample,
d) performing a biochemical analysis to produce a molecular profile of said isolated cell or said number of isolated cells, said molecular profile comprises a plurality of molecular values, and
e) producing a modified digital image comprising said digital image obtained in step a) together with one or more of said molecular values produced in step d) to perform said molecular histological analysis.

12. The method according to claim 11, wherein said biochemical analysis is a gene expression analysis based on PCR amplification, preferably a real-time PCR.

13. The method according to claims 11 to 12, wherein said biochemical analysis is an immunoassay.

14. A method to identify the location of characteristic cells within a multicellular sample in order to perform a molecular histological analysis, said method comprises
a) providing a digital image of said multicellular sample together with molecular profiles of cells or number of cells within said multicellular sample, each molecular profile comprises a plurality of molecular values and is linked to a region of interest within said digital image corresponding to the respective cell or number of cells,
b) highlighting in a modified digital image those cells within said multicellular sample that fulfill a defined molecular criteria,
wherein said molecular profiles are obtained by isolating a cell or a number of cells from said multicellular sample.

15. A computer program to generate a plurality of modified digital images from the digital image of a multicellular sample, said digital images are modified with one or more molecular value of respective cells or number of cells within said multicellular sample, said computer program comprises
a) loading said digital image of said multicellular sample and a plurality of molecular values of cells or number of cells from said multicellular sample, each molecular value is linked to a region of interest within said digital image corresponding to the respective cell or number of cells, and
b) generating a modified digital image comprising said digital image and a number of selected molecular values,
wherein said molecular values are obtained by isolating a cell or a number of cells from said multicellular sample.

16. A method for managing molecular histological experiments of a multicellular sample, said method comprises
a) loading a digital image of said multicellular sample into a software application,
b) defining regions of interest within said digital image that are linked to corresponding positions of cells within said multicellular sample, and
c) loading a plurality of molecular profiles of cells or number of cells from said multicellular sample into said software application, each molecular profile comprises a plurality of molecular values,
wherein said software application is structured such that said plurality of molecular profiles loaded in step c) are linked to the corresponding region of interest defined in step b) within said digital image loaded in step a) and
wherein said molecular profiles are obtained by isolating a cell or a number of cells from said multicellular sample.

17. The method according to claim 16, wherein a modified digital image of said multicellular sample is produced in order to perform a histological analysis of said multicellular sample.

18. The method according to claims 16 to 17, wherein said software application is a database, an image-based application or a combination thereof.

19. A computer program to identify the location of characteristic cells within a multicellular sample in order to perform a molecular histological analysis, said computer program comprises
a) a first application to load a digital image of multicellular sample, to define regions of interest within said digital image that are linked to corresponding positions of cells within said multicellular sample and to save said digital image together with said regions of interest as a first image file,
b) a second application to combine said first image file with a plurality of molecular profiles comprising a plurality of molecular values and to save said first image file together with said plurality of molecular profiles as a second image file, wherein said plurality of molecular profiles are linked to the corresponding region of interest within said digital image, and
c) a third application to extract modified digital images from said second image file according to user-defined molecular criteria, wherein said modified digital images highlight cells that fulfill said user-defined molecular criteria,
wherein said molecular profiles are obtained by isolating a cell or a number of cells from said multicellular sample.

20. The computer program according to claim 19, wherein said computer program is an image-based application, preferably a TIFF-application.

21. A database for managing molecular histological experiments of a multicellular sample, said database comprises
a) a cell table comprising cell numbers together with the corresponding position within said multicellular sample,
b) a molecular value table comprising molecular profiles of cells within said multicellular sample, each molecular profile comprises a plurality of molecular values, and
c) a digital image table comprising digital images of said multicellular sample and coordinates of cells within said digital image,
wherein said cell table is linked with said molecular value table and with said digital image table such that each cell of said multicellular sample is linked to the corresponding coordinates within the respective digital image and to the corresponding molecular profile,
wherein said database is designed such that a modified digital image based on a digital image from said digital image table highlighting cells fulfilling a plurality of user-defined molecular criteria is producible and
wherein said molecular profiles are obtained by isolating a cell or a number of cells from said multicellular sample.
